# EUROPEAN PATENT APPLICATION

(11) **EP 2 255 782 A1**
(43) Date of publication of application: **01.12.2010**
(21) Application number: 09161023.8
(22) Date of filing: 25.05.2009
(51) Int. Cl.: A61K 8/97, A61Q 7/00

(54) **Composition for the prevention or treatment of hair loss, and method of preparation**

(71) Applicant: Keune Haircosmetics Manufacturing B.V., 3762 EA Soest (NL)
(72) Inventor: Keune, George, 3762 EA, Soest (NL)
(74) Representative: Van den Heuvel, Henricus Theodorus

(57) **Abstract**

The invention relates to an improved composition for the prevention or treatment of hair loss, and a method for the preparation of such a composition. The composition comprises an effective amount of panax ginseng root extract, preferably a red ginseng extract. Optionally, ginseng may be combined with other active components such as quaternium-51, 3-aminopropane sulfonic acid, biotin, caffeine and protein-bound minerals, preferably formulated as a liposomal composition.

## Description

The invention relates to a composition for the prevention or treatment of hair loss. The invention also relates to a method for the preparation of such a composition.

Human hair growth occurs in distinct phases, in particular a growth phase (anagen), a transition phase (catagen) and a resting phase (telogen). The anagen phase is characterised by the growth of a hair from a hair follicle cell. After approximately 1000 days, the hair follicle enters the catagen phase, resulting in a loosely bound hair which ultimately results in the loss of the hair. Typically, approximately 80 to 100 hairs fall out daily from the scalp. After that, the telogen phase is entered, which may be reactivated after a certain period (typically 100 days) to re-enter the anagen phase to produce a new hair. When the amount of hair falling out exceeds the amount of new hairs produced, a netto hair loss is occurring, which may be reversible or irreversible.

Reversible hair loss includes hair loss as a result of illness, malnutrition or certain medicines. Androgenic hair loss, the conversion of terminal hair into vellus hair, is the classical form of hair loss that has been classified up to now as largely irreversible. It may start in early manhood (at around 17) and may be complete by about age 50. The two most important biologically active forms of the androgens are testosterone and 5-alpha-dihydrotestosterone derived from it. An enzyme, 5-alpha-reductase, converts testosterone into dihydrotestosterone, which is the active hormone form for influencing the growth of hair. Androgens, which act on the hair and body hair, are soloists in the hormone orchestra. In older men hair loss due to old age, which predominantly involves follicle transformations of terminal follicles into intermediary follicles and vellus follicles, in both cases leads to formation of a further diffuse bare region in the still existing hair. In this stage of old age the hair requires special completely effective and specially suited care. Disturbances, which lead to a destruction of the hair organ, cause permanent baldness.

It is an object of the invention to provide a means for preventing or treating hair loss.

The invention provides a composition for the prevention or treatment of hair loss, wherein the composition comprises an effective amount of panax ginseng root extract. Panax ginseng root extract was shown to be an effective means for preventing as well as treating hair loss. Panax ginseng includes extracts from the roots of *P. quinquefolius* (American ginseng) as well as *Panax ginseng* (Asian ginseng). Preferably, the composition is suitable for application to the hair and/or scalp. Liquid or gel-like compositions are preferred for easy and effective application of the composition over the area to be treated. Panax ginseng induces an earlier telogen to anagen conversion with respect to the well-known hair growth compound minoxidil, as well as control experiments. Also, an increased expression of the growth factor gene of VEGF (vascular endothelial growth factor) is observed. Other effects are an increased mean hair length (shown in-vitro), and an increased depth and size of the hair follicles.

It is preferred if the composition comprises at least 0.00001 % w/w in dry weight of panax ginseng root extract, preferably from 0.00001-1%, most preferably from 0.00001-0.04% w/w dry weight. The composition may further comprise solvents, cosolvents, solubizers and other ingredients. The panax ginseng extract may be used as a commercially available stabilized form dissolved in a water/butylenes glycol mixture, which typically comprises approximately 60% butylene glycol, approximately 40% water, and 0.2% dry weight of red panax ginseng root extract. A stabilized solution makes it easier to dose and process the panax ginseng.

Preferably, the panax ginseng root extract is a red ginseng extract. The stimulation of hair growth was markedly increased with respect to white ginseng extracts, which supports the notion that Red Ginseng induces early onset of the anagen phase and stimulates hair growth. White and red ginseng refer to the way the ginseng root is processed. White ginseng is peeled and sun-dried. In contrast, red ginseng is not peeled and cured using steam, followed by drying. The resulting ginseng product has a reddish-brown colour. It was found that in the combinations of active compounds described below, compositions using red ginseng consistently performed better against hair loss than similar compositions using white ginseng instead. A particularly compound of interest found in relatively high abundance in red ginseng extracts, and having a high activity against hair loss, is ginsenoside Rg2, a derivative of 20(S)-protopanaxatriol. Preferably, the composition comprises a combination of panax ginseng root extract and an effective amount of quaternium-51. Quaternium-51 (CAS 1463-95-2, 2-[2-[(5-bromo-2-pyridyl)amino]vinyl]-1-ethyl-6-methylpyridinium iodide), is commercially available under the brand name Takanal. Quaternium-51 is believed to activate cell metabolism, which results in a stimulation of hair growth. Preferably, quaternium-51 is used in at least 0.0001 % w/w. most preferably 0.0001-1% w/w. The combination of panax ginseng and quaternium-51 shows a synergistic effect: the combination promotes the regeneration of hair and prevents hair from shedding. As a bonus effect, the combination is also particularly effective in controlling dandruff.

In another preferred embodiment, the composition comprises a combination of panax ginseng root extract and an effective amount of 3-aminopropane sulfonic acid. 3-aminopropane sulfonic acid is also known as homotaurine. 3-aminopropane sulfonic acid slows down excessive hair loss and stimulates hair renewal and regeneration. Also, it regulates seborrhea, which is often associated with the alopecic process. These effects are amplified when used in combination with panax ginseng extract, and result in a decrease in the number of hairs falling out, a stimulation of hair growth and a reduction of hair loss associated with seborrhoea. Derived salts or esters of aminopropane sulfonic acid may also be used. Preferably, 3-aminopropane sulfonic acid is present in at least 0.0003% w/w, most preferably from 0.0003-1.5 % w/w. The combination of 3-aminopropane sulfonic acid and panax ginseng shows a synergistic increase in the prevention of hair loss. A suitable commercially available formulation of 3-aminopropanesulfonic acid is a solution comprising approximately 3% by weight of 3-aminopropane sulfonic acid, 25% by weight of hydrolysed soy protein, 60% by weight of water, 9 % by weight of glycerin and 3% by weight of sodium chondroitin sulphate. Such a stabilized solution may be added to achieve the desired amount of 3-aminpropane sulfonic acid. The hydrolysed soy protein and sodium chondroitin sulphate also contribute to the hair growth.

It is advantageous if the composition comprises a combination of panax ginseng root extract and an effective amount of biotin. Preferably, the composition comprises at least 0.0001 % w/w biotin, preferably from 0.0001 to 1 % w/w. The combination of biotin and panax ginseng shows a synergistic increase in the prevention of hair loss. Biotin improves keratinisation and strengthens the anchoring of the bottom of the hair in the scalp. When used in combination with panax ginseng root extract, these known hair strengthening and hair anchoring effects of biotin were improved. These effects result in a decrease in the number of hairs falling out.

Preferably, the composition comprises a combination of panax ginseng root extract and an effective amount of a vasodilator, preferably caffeine. The combination of panax ginseng and an additional vasodilator shows a synergistic increase in the prevention of hair loss. A vasodilator increases blood supply to the hair organ, thus increases its supply of nutrients, which results in a prolonged growth cycle of a hair. Preferably, the composition comprises at least 0.01 % w/w, preferably 0.01 to 10 % w/w of caffeine or an amount of another vasodilator or mixture of vasodilators of equivalent vasodilating activity, for instance menthol, tocopherols and their esters, nicotinic acid and its esters, nicotamide and camphor,.

In a preferred embodiment, the composition comprises a combination of panax ginseng root extract and an effective amount of protein-bound minerals. Preferably, the composition comprises at least 0.0001 % w/w dry weight of protein-bound minerals, more preferably from 0.0001 to 1 % w/w. Protein-bound minerals were shown to have an advantageous effect on hair growth. Preferably, the protein bound minerals comprise silicon, zinc, copper, iron and magnesium within physiologically acceptable concentrations. These proteins and minerals provide nutrients for hair growth. Silicon plays an important role in the formation of hair, skin and nails. Zinc is an important nutrient necessary for wound healing and development of new cells. Copper is important in formation of disulfide crosslinkages in keratinization, promoting hair growth. Iron aids in proper oxygenation of tissues to maintain basic life functions. It is essential for cell maturation and protein formulation. Magnesium is a major component in the enzymatic reactions of carbohydrates, proteins and energy metabolism. A preferred source of protein-bound minerals are yeast protein extracts, most preferably derived from yeast of the genus *Saccharomyces.*

Most preferably, the composition comprises a combination of panax ginseng root extract, an effective amount of biotin and an effective amount of a vasodilator, preferably caffeine. This combination of active ingredients shows an additional synergistic effectively in the prevention of hair loss. The minimum effective amounts are the same as those for the combinations mentioned above. Even better results are obtained using a composition comprising a combination of panax ginseng root extract, an effective amount of biotin and an effective amount of a vasodilator, preferably caffeine, and protein-bound minerals.

It is preferred if the composition is a liposomal composition, preferably comprising liposomes of phospholipids. It was shown that the effectiveness of panax ginseng root extract against hair loss was increased when formulated as a liposomal composition. Combinations of panax ginseng root extract and other active ingredients as stated above were also shown to benefit from a liposomal composition. It is postulated the liposomes act as a delivery system for the active ingredients in the panax ginseng root extract. In addition, a lipsomal composition improves the stability of the composition in water or an aqueous solution or a water/alcohol mixture. Also, the solubility of components in the panax ginseng extract are increased.

Liposomes are spherical vesicles formed by double layers of lipids (bilayers), which may encapsulate active substances in solution. The particle size of the liposomes in the present invention is preferably from 70 to 300 nm (diameter), more preferably 150 to 200 nm. Suitable phospholipids may be retrieved from various sources. Lecithin is a preferred source of phospholipids for the panax ginseng root extract compositions according to the invention. The phospholipids preferably comprise at least 73 weight %; phosphatidyl choline, preferably 73 - 79 % w/w. In addition, the phospholipids may comprise other phospholipids as well as other components.

Preferably, the composition comprises liposomes of phospholipids, wherein the weight ratio of phospholipids to the dry weight of the panax ginseng root extract is in the range from 5:1-1000:1, more preferably from 50:1 to 1000:1. In such a ratio an optimum effect is achieved.

It is preferred if the composition is a liposomal composition comprising a combination of panax ginseng root extract and an effective amount of at least one further active component selected from the list consisting of quaternium-51, 3-aminopropane sulfonic acid, biotin , caffeine and protein-bound minerals. These compositions have a synergetic effect when combined with panax ginseng, as stated above. This synergetic effect is amplified in a liposomal composition. If other active ingredients are included, in particular quaternium-51, 3-aminpropanesulfonic acid, caffeine and/or biotin, the ratio of said active ingredients including panax ginseng with respect to the phospholipids is preferably in the range from 50:1 to 1000:1.

Most preferably, the composition is a liposomal composition comprising a combination of panax ginseng root extract, an effective amount of biotin , an effective amount of a vasodilator, preferably caffeine, and protein-bound minerals. This combination has the most optimal effect. The ratio of said combination of active ingredients with respect to the phospholipids is preferably in the range from 50:1 1 to 1000:1.

It is preferred if the composition comprises a solvent suitable for topical application, preferably water or an aqueous solution, more preferably a water-alcohol mixture. Such a composition is easily applied, highly tolerated by the scalp and very suitable to spread the active ingredients, in particular panax ginseng root extract, over the scalp to be treated. Preferably, the composition comprises from 70-95% w/w of solvent.

Most preferably, the composition is formulated as a lotion, a tonic, a gel or an elixir. The main difference of these formulations is the viscosity of the formulation. Ranging from the most viscous to the most fluid formulation, the order is gel>elixir>lotion>tonic.

The invention also provides a method for the preparation of a composition for the prevention or treatment of hair loss, comprising the addition of an effective amount of panax ginseng root extract to an aqueous solvent, preferably a water-alcohol mixture. The ingredients may be mixed by conventional mixing means. Additional solubizers may be needed in order to ensure a stable solution of the panax ginseng root extract. Preferably, the active ingredients are added as a solution for easy homogeneous mixing.

Preferably, the method comprises the addition of phospholipids to an aqueous solvent to form a liposomal mixture, followed by the addition of at least an effective amount of panax ginseng root extract to the liposomal mixture. Preferably, the mixing is done by a high pressure homogeniser. Mixing in this way ensures liposomes encapsulating at least part of the panax ginseng root extract are formed in the aqueous solution.

Most preferably, also an effective amount of at least one further active component is added to the liposomal mixture, selected from the list consisting of quaternium-51, 3-aminopropane sulfonic acid, biotin, caffeine and protein bound minerals. These compounds are also included in the liposomes.

The invention will now be further elucidated by the following non-limiting examples.

### Example 1: Hair lotion

Table 1 shows a formulation of a hair lotion according to the invention, suitable for application to the scalp. As active ingredients, Lecithin, Planoxia RG (Radiant), Quaternium-51 / Takanal (Ikeda), Capigen™ PH (Sederma), Caffeine, Biotin and Protein bound minerals were included.

Lecithin is the main source for phospholipids forming the liposomes in the formulation. Lecithin was obtained from Phospholipid GmbH as a composition consisting of 75% lecithin and 25% ethanol. The phospholipid composition used comprised approximately 78% Phosphatidyl choline, 3% Lysophosphatidyl choline, 4% Cephaline and 1.5% Phosphatidic acid.

Planoxia RG, obtained from Radiant, is a stabilized formulation of panax ginseng root extract, comprising approximately 40% water, 60% butylene glycol and 0.2 % Panax Ginseng Root Extract by weight.

Quaternium-51 (Takanal, 2-[2-[(5-bromo-2-pyridyl)amino]vinyl]-1-ethyl-6-methylpyridinium iodide) was obtained from Ikeda.

Capigen™ PH was obtained from Sederma, and is a stabilized solution of 3-Aminopropane Sulfonic Acid (homotaurine). The solution comprises 3% w/w 3-Aminopropane Sulfonic Acid, 25% w/w hydrolyzed soy protein, 3% w/w Sodium Chondroitin Sulfate, 9 % w/w glycerine and 60% w/w water.

Protein-bound minerals were obtained from yeast extracts. The mixture comprises 0.09 % w/w Saccharomyces/Silicon Ferment, 2.61% w/w Saccharomyces/Magnesium Ferment, 0.15% w/w Saccharomyces/Copper Ferment, 0.84% w/w Saccharomyces/Iron Ferment, 0.33% w/w Saccharomyces/Zinc Ferment and 95.98% w/w water.

Caffeine, biotin and all other ingredients were of pharmaceutical quality, obtained from generic resellers.

**Table 1: Hair lotion formulation**

| **Ingredient (lotion)** | **Percentage** **% (^{w}/_{w})** |
|---|---|
| Alcohol denat. | 15 |
| Glycerin | 0,3 |
| Lecithin | 0,5 |
| Planoxia RG (Radiant) | 2 |
| Quaternium-51 / Takanal (Ikeda) | 0,002 |
| Capigen™ PH (Sederma) | 2 |
| Caffeine | 0,1 |
| Biotin | 0,002 |
| Mixture of Protein bound minerals | 0,4 |
| Propylene Glycol | 0,05 |
| Menthol | 0,15 |
| Sodium Benzoate | 0,2 |
| PEG-40 Hydrogenated Castor Oil | 0,3 |
| Parfum/Fragrance | 0,1 |
| Citric Acid | 0,01 |
| Aqua/Water | up to 100% |

First, a liposome phase was prepared by adding phospholipids to water and using a high pressure homogenizer operated twice at 600 bar. Subsequently, a liquid premix of the other desired effective ingredients was added and mixed using the high pressure homogenizer at 600 bar. The resulting liposomal mixture was added to a water-alcohol mixture, and a preservative (sodium benzoate), perfume (any may be added), and pH buffer (citric acid) and optionally other solubilizers (hydrogenated castor oil) were added in order to achieve a stable solution.

The stable hair lotion was easy to apply to the scalp, and was reported to at least slow the pace of hair loss in some test persons. The hair lotion, as well as the gel, tonic and elixir formulations presented below, are reported to stimulate hair growth, as illustrated by the in vitro tests.

In another formulation, a hair gel was prepared. In addition to the active ingredients against hair loss as discussed above, a carbomer was added as a thickener, along with triethanolamine to stabilize the carbomer and a VP/VA copolymer as a hair fixative. The gel is significantly more viscous than the lotion described above, and as an additional feature also fixes the hair. This formulation does not contain liposomes.

**Table 2: Hair gel formulation**

| **Ingredient (gel)** | **Percentage** **% (^{w}/_{w})** |
|---|---|
| Alcohol denat. | 20 |
| Carbomer | 0,7 |
| Triethanolamine | 1 |
| VPNA Copolymer | 1 |
| Planoxia RG (Radiant) | 2 |
| Quaternium-51 / Takanal (Ikeda) | 0,0001 |
| Capigen™ PH (Sederma) | 0,1 |
| Caffeine | 1 |
| Biotin | 0,01 |
| Mixture of Protein bound minerals | 0,2 |
| PEG-40 Hydrogenated Castor Oil | 1 |
| Parfum/Fragrance | 0,5 |
| Aqua/Water | up to 100% |

### Example 3: Elixir

Also, a composition according to the invention was formulated as a hair elixir. The elixir has a viscosity between the gel and lotion formulations described above. This formulation does not contain liposomes. Hydroxyethylcellulose was used as a thickener agent.

**Table 3: Hair elixir formulation**

| **Ingredients (elixir)** | **Percentage** **% (^{w}/_{w})** |
|---|---|
| Alcohol denat. | 25 |
| Hydroxyethylcellulose | 0,5 |
| Planoxia RG (Radiant) | 6 |
| Quaternium-51 / Takanal (Ikeda) | 0,001 |
| Capigen™ PH (Sederma) | 2 |
| Caffeine | 0,5 |
| Biotin | 0,01 |
| Mixture of Protein bound minerals | 1 |
| PEG-40 Hydrogenated Castor Oil | 1 |
| Parfum/Fragrance | 0,5 |
| Citric Acid | 0,01 |
| Aqua/Water | up to 100% |

### Example 4: Hair tonic

The hair tonic is less viscous than the hair lotion presented here. The current formulation does not comprise liposomes.

**Table 4: Hair Tonic formulation**

| **Ingredients (tonic)** | **Percentage** **% (^{w}/_{w})** |
|---|---|
| Alcohol denat. | 50 |
| Planoxia RG (Radiant) | 2 |
| Quaternium-51 / Takanal (Ikeda) | 0,005 |
| Capigen™ PH (Sederma) | 3 |
| Caffeine | 1 |
| Biotin | 0,01 |
| Mixture of Protein bound minerals | 1 |
| Menthol | 0,15 |
| PEG-40 Hydrogenated Castor Oil | 0,15 |
| Parfum/Fragrance | 0,1 |
| Aqua/Water | up to 100% |

### Example 5: in-vitro test

The hair lotion, as well as the gel, tonic and elixir formulations presented above, are reported to stimulate hair growth, which is illustrated by in vitro tests performed on hair follicle cells.

Three solutions O, P and Q related to the lotion formulation in table 1 were prepared for in-vitro testing. The three different solutions contained different cocktails of active and non-active ingredients, in order to test the effect on hair follicle growth. The solutions only make up for 10% of the total formulations used.

**Table 2: In Vitro-tests formulations**

| | O | P | Q |
|---|---|---|---|
| Hydrogenated Lecithin | 0,500 | 0,500 | 0,500 |
| Sodium Benzoate | 0,010 | 0,010 | 0,010 |
| **Mixture of Protein bound minerals** | 0,410 | 0,410 | 0,410 |
| **Capigen PH** | 2,000 | 4,090 | |
| **Takanal** | 0,002 | 0,002 | |
| **Biotin** | 0,002 | 0,002 | |
| **Caffeine** | 0,100 | 0,100 | |
| **Planoxia RG** | 2,000 | | |
| Aqua/water | up to 10% | up to 10% | up to 10% |

A model of in vitro human hair follicle culture, directly derived from the widely validated standard conditions described by Philpott (Philpott M.P., Sanders D., Westgae.E., and Kealey T. (1994) Human hair growth in vitro: a model for the study of hair follicle biology. J Dermatol Sci Jul;7 Suppl:S55-72), allows testing compounds capable of modulating stimulation of hair growth (regrowth of the hairs), inhibition of hair growth (anti-regrowth of the hairs), increase of culture survival (anti-apoptose, anti-loss), increase of the hair root diameter, pigmentation of hair follicles, which are an indication of the efficacy of the combinations of compounds for the prevention and treatment of hair loss.

This model consists in a whole isolated organ and represents a "natural" coculture model of normal cutaneous differentiated cells (keratinocytes, but also fibroblasts, melanocytes and original cells). Obviously, this model does not include blood vessels and this limits the detection of products active on scalp via blood circulation.

In the present study, the effects of O, P and Q on hair follicle elongation (after 4, 7, 11 and 14 days of culture) and follicle survival were researched.

### Biological model

- Cellular model: Hair follicles microdissected from a human skin fragment.
- Culture condition: 37 °C, 5% CO₂.
- Culture medium: E William's medium supplemented with Penicillin/streptomycin, 50 U/ml - 50 µg/ml, L-glutamine 2 mM, Insulin 10 µg/ml, Hydrocortisone 8x 10⁻⁹M.

### Hair follicle growth

The hair bulbs were cultured in culture medium containing or not (control) the test compounds. The hair follicles growth was followed for 20 days with length measurements at day 0 (D0), day 4 (D4), day 11 (D11) and day 14 (D14) and photographs at D0, D4, D7, D11, D14, D15, D18, D19 and D20. For each condition, an excess of hair bulbs was isolated to reach the target-value of 12 high quality hair follicles.

After several days of growth, when follicles begin to degenerate and enter into apoptotic phases, photographs were taken and images showing the beginning of degeneration of the follicles after 14 days of culture were counted and the number of apoptotic follicles was compared to control follicles. Percentage of apoptosis per condition was calculated by counting the number of apoptotic hair follicles (showing a beginning of degeneration of the follicles) versus the total number of bulbs.

Hair follicles growth was normal and in proportion to results usually obtained in the laboratory. The treatment with O at 0.8 mg/ml significantly increased hair growth after 4 and 7 days of culture, respectively 125% and 119% compared to control, and also significantly better than P and Q.

In these experimental conditions, only formulation O showed a stimulating effect on hair growth. As O is the only formulation including panax ginseng root extract, these results show the positive influence of panax ginseng root extract, in particular when formulated with liposomes and the mentioned other active ingredients.

## Claims

1. Composition for the prevention or treatment of hair loss, wherein the composition comprises an effective amount of panax ginseng root extract.

2. Composition according to claim 1, **characterised in that**
the panax ginseng root extract is a red ginseng extract.

3. Composition according to claim 1 or 2, **characterised in that**
the composition comprises a combination of panax ginseng root extract and an effective amount of quaternium-51.

4. Composition according to any of the preceding claims, **characterised in that**
the composition comprises a combination of panax ginseng root extract and an effective amount of 3-aminopropane sulfonic acid.

5. Composition according to any of the preceding claims, **characterised in that**
the composition comprises a combination of panax ginseng root extract and an effective amount of biotin.

6. Composition according to any of the preceding claims, **characterised in that**
the composition comprises a combination of panax ginseng root extract and an effective amount of a vasodilator, preferably caffeine.

7. Composition according to any of the preceding claims, **characterised in that**
the composition comprises a combination of panax ginseng root extract and an effective amount of protein-bound minerals.

8. Composition according to any of the preceding claims, **characterised in that**
the composition comprises a combination of panax ginseng root extract, an effective amount of biotin and an effective amount of a vasodilator, preferably caffeine.

9. Composition according to any of the preceding claims, **characterised in that**
the composition is a liposomal composition, preferably comprising liposomes of phospholipids.

10. Composition according to claim 9, **characterised in that**
the composition comprises liposomes of phospholipids, wherein the weight ratio of phospholipids to the dry weight of the panax ginseng root extract is in the range from 50:1 to 1000:1.

11. Composition according to claim 9 or 10, **characterised in that**
the composition is a liposomal composition comprising a combination of panax ginseng root extract and an effective amount of at least one further active component selected from the list consisting of quaternium-51, 3-aminopropane sulfonic acid, biotin, caffeine and protein-bound minerals.

12. Composition according to any of the claims 9-11, **characterised in that**
the composition is a liposomal composition comprising a combination of panax ginseng root extract, an effective amount of biotin and an effective amount of a vasodilator, preferably caffeine.

13. Composition according to any of the preceding claims, **characterised in that**
the composition comprises a solvent suitable for topical application, preferably an aqueous solution, more preferably a water-alcohol mixture.

14. Composition according to any of the preceding claims, **characterised in that**
the composition is formulated as a lotion, an elixir, a gel or a tonic.

15. Method for the preparation of a composition for the prevention or treatment of hair loss, comprising the addition of an effective amount of panax ginseng root extract to an aqueous solvent, preferably a water-alcohol mixture.
